# EUROPEAN PATENT APPLICATION

(11) **EP 3 991 776 A1**
(43) Date of publication of application: **04.05.2022**
(21) Application number: 19935159.4
(22) Date of filing: 28.06.2019
(51) Int. Cl.: A61M 25/09

(54) **GUIDE WIRE**

(71) Applicant: ASAHI INTECC CO., LTD., Seto-shi, Aichi 489-0071 (JP)
(72) Inventor: SAIKI Shunsuke, Seto-shi, Aichi 489-0071 (JP)
(74) Representative: Winter, Brandl - Partnerschaft mbB
(86) International application number: PCT/JP2019/025852
(87) International publication number: WO 2020/261542

(57) **Abstract**

A guide wire includes a core shaft, and a coil body wound around a distal end side of the core shaft, and a distal tip to which a distal end portion of the core shaft and a distal end portion of the coil body are fixed. The distal end portion of the core shaft has a rectangular transverse sectional shape, and an aspect ratio of the rectangle is greater than or equal to 1:1 and less than or equal to 1.08:1.

## Description

### TECHNICAL FIELD

The disclosed embodiments relate to a guide wire.

### BACKGROUND ART

There are known guide wires used for inserting a medical device such as a catheter into a blood vessel. In such a guide wire, to improve selectivity for blood vessels and smoothly lead the guide wire to a target site in the blood vessel, a shape such as a small curve is provided to a distal end portion of the guide wire in some cases. Hereafter, formation of a curved shape or the like is also referred to as "shaping". For example, Patent Literature 1 and Patent Literature 2 disclose a guide wire in which a flat portion is provided to a distal end portion of a core shaft to facilitate shaping of the distal end portion (to improve a shaping performance).

### CITATION LIST

### Patent Literature

Patent Literature 1: JP 2013-544575 W
Patent Literature 2: U.S. Patent No. 4846174

### SUMMARY OF INVENTION

### TECHNICAL PROBLEM

Incidentally, to improve the selectivity for blood vessels, not only the shaping performance of the distal end portion of the guide wire but also rotation followability for transmitting a rotational operation of a butt portion for the guide wire to the distal end side are required. A transverse sectional shape on the distal end portion of the core shaft is important for achieving both the shaping performance and the rotation followability. In this regard, it is described that the distal end portion of the core shaft has a square transverse sectional shape in the guide wire of Patent Literature 1. However, in the manufacture of the guide wire, the transverse sectional shape on the distal end portion of the core shaft can hardly be formed in a perfect square without error, and the technology described in Patent Literature 1 has had a problem that any permissible error for maintaining the performance is not taken into consideration.

In addition, it is described that the distal end portion of the core shaft has a rectangular transverse sectional shape in the guide wire described in Patent Literature 2. However, the technology described in Patent Literature 2 has had a problem that any preferable length-to-width ratio, and any permissible error for maintaining the performance are not taken into consideration. Such a problem is not limited to a vascular system, and is common to guide wires to be inserted into various organs in a human body, such as a lymphatic system, a biliary system, a urinary system, a respiratory system, a digestive system, a secretory gland, and a reproductive organ.

The disclosed embodiments have been made to solve at least a part of the aforementioned problems, and an object of the disclosed embodiments is to achieve both the shaping performance and the rotation followability in the guide wire.

### SOLUTION TO PROBLEM

The disclosed embodiments were made to solve at least a part of the aforementioned problems, and can be achieved as the following aspects.
(1) According to one aspect of the disclosed embodiments, a guide wire is provided. This guide wire includes a core shaft, a coil body wound around a distal end side of the core shaft, and a distal tip to which a distal end portion of the core shaft and a distal end portion of the coil body are fixed, wherein the distal end portion of the core shaft has a rectangular transverse sectional shape, and an aspect ratio of the rectangle is greater than or equal to 1:1 and less than or equal to 1.08:1.
   According to this configuration, in the guide wire, the distal end portion of the core shaft has the rectangular transverse sectional shape and the rectangle is almost square with the aspect ratio of greater than or equal to 1:1 and less than or equal to 1.08:1, and therefore both the shaping performance and the rotation followability of the guide wire can be achieved. In addition, when the aspect ratio of the rectangle is set to be greater than or equal to 1:1 and less than or equal to 1.08:1, the shaping performance and the rotation followability of the guide wire can be maintained while facilitating manufacture of the guide wire.
(2) In the guide wire according to the above aspect, the core shaft may include the distal end portion having a prismatic shape, a first intermediate portion adjacent to a proximal end side of the distal end portion and having a rectangular transverse sectional shape on the distal end and a circular transverse sectional shape on the proximal end in which the transverse sectional shape gradually changes from the rectangular shape to the circular shape from the distal end toward the proximal end, and a second intermediate portion adjacent to the proximal end side of the first intermediate portion and having a circular transverse sectional shape over its entire length.
   According to this configuration, the core shaft includes the first intermediate portion having the transverse sectional shape gradually changing from the rectangular shape to the circular shape from the distal end toward the proximal end, between the prismatic (i.e. rectangular transverse sectional shape) distal end portion and the second intermediate portion having the circular transverse sectional shape. Thereby, it is possible to smoothly change a rigidity and an outer shape from the distal end portion, to the first intermediate portion, and to the second intermediate portion. As a result, stress concentration at a specific position of the core shaft can be suppressed, and kink resistance of the guide wire can be improved during shaping and use of the guide wire.
(3) In the guide wire according to the above aspects, a sectional area of a transverse section at any position of the distal end portion may be equal to a sectional area of a transverse section on a distal end of the second intermediate portion.
   According to this configuration, in the core shaft, the sectional area of the transverse section on the distal end portion is equal to the sectional area of the transverse section on the distal end of the second intermediate portion. Thereby, stress concentration at a specific position of the core shaft can be suppressed, and kink resistance of the guide wire can be improved during shaping and use of the guide wire.
(4) In the guide wire according to the above aspects, a curved portion in which the core shaft and the coil body are curved with respect to an axial direction of the guide wire may be provided on the distal end side of the guide wire.
   According to this configuration, a curved portion having the curved core shaft and coil body is provided on the distal end side of the guide wire. Herein, since the distal end portion of the core shaft has the rectangular transverse sectional shape with the aspect ratio of greater than or equal to 1:1 and less than or equal to 1.08:1, the curved portion can be easily provided on the distal end side of the guide wire.
(5) The guide wire according to the above aspects may further include a strand composed of a plurality of wires twisted together, which is arranged alongside the core shaft inside the coil body, and whose distal end portion fixed to the distal tip, and a fixation portion provided on the second intermediate portion of the core shaft, to which a proximal end portion of the strand, the core shaft, and the coil body are fixed.
   According to this configuration, the guide wire further include the strand composed of a plurality of wires twisted together, and therefore can be configured such that the strand and the core shaft integrally move on the distal end side of the guide wire during use of the guide wire. As a result, a guide wire excellent in flexibility and restorability against bending can be provided.
(6) In the guide wire according to the above aspects, a curved portion in which the strand, the core shaft, and the coil body are curved with respect to the axial direction of the guide wire may be provided on the distal end side of the guide wire.
   According to this configuration, the curved portion where the strand, the core shaft, and the coil body are curved is provided on the distal end side of the guide wire. Herein, since the distal end portion of the core shaft has the rectangular transverse sectional shape with the aspect ratio of greater than or equal to 1:1 and less than or equal to 1.08:1, the curved portion can be easily provided on the distal end side of the guide wire.

Note that the disclosed embodiments can be achieved in various aspects and in a form of e.g. a medical device such as a guide wire, a core shaft, and a catheter using a core shaft, as well as a manufacture method therefor.

### BRIEF DESCRIPTION OF DRAWINGS

Fig. 1 is an explanatory diagram illustrating a configuration of a guide wire according to the first embodiment.
Fig. 2 is an explanatory diagram illustrating a sectional configuration on a distal end side of the core shaft.
Fig. 3 is an explanatory diagram illustrating configurations of samples of guide wires used for a rotation followability test.
Fig. 4 is an explanatory diagram of a test method for the rotation followability test.
Fig. 5 is an explanatory diagram of a test result for the rotation followability test.
Fig. 6 is an explanatory diagram of evaluation results for the rotation followability test.
Fig. 7 is an explanatory diagram illustrating a configuration of a guide wire according to the second embodiment.
Fig. 8 is an explanatory diagram illustrating a configuration of a guide wire according to the third embodiment.
Fig. 9 is an explanatory diagram illustrating a sectional configuration on a distal end side of a core shaft according to the fourth embodiment.
Fig. 10 is an explanatory diagram illustrating a sectional configuration on a distal end side of a core shaft according to the fifth embodiment.
Fig. 11 is an explanatory diagram illustrating a configuration of a guide wire according to the sixth embodiment.
Fig. 12 is an explanatory diagram illustrating a configuration of a guide wire according to the seventh embodiment.

### EMBODIMENTS OF THE INVENTION

### <First Embodiment>

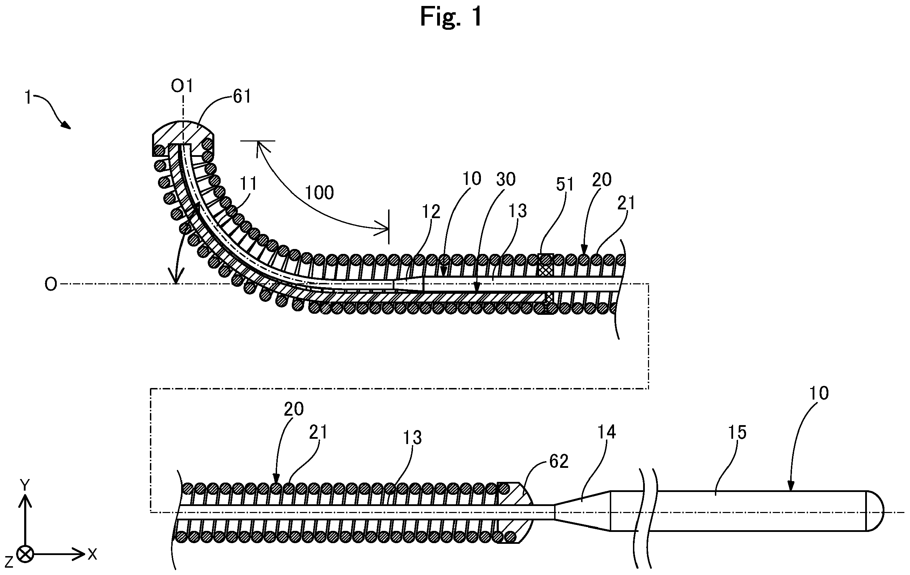

Fig. 1 is an explanatory diagram illustrating a configuration of a guide wire 1 according to the first embodiment. The guide wire 1 is a medical device used for inserting a catheter into a blood vessel or a digestive organ, and includes a core shaft 10, a coil body 20, a strand 30, a fixation portion 51, a distal tip 61, and a proximal fixation portion 62. A curved portion 100 in which the guide wire 1 is curved is provided on a distal end portion of the guide wire 1. In the guide wire 1 according to the first embodiment, a distal end portion of the core shaft 10 has a configuration described later, so that both a shaping performance and a rotation followability of the guide wire 1 can be achieved. The shaping performance means easiness of shaping. In addition, the rotation followability means a performance to rotate the distal end side of the guide wire 1 following the rotation on the proximal end side of the guide wire 1. In other words, the rotation followability refers to a performance to transmit the rotational operation of the guide wire 1 on the proximal end side to the distal end side.

In Fig. 1, a central axis of the guide wire 1 is represented by an axis line O, and a central axis of the curved portion 100 is represented by an axis line O1. In an example of Fig. 1, the axis lines O and O1 coincide with a central axis of the core shaft 10 and a central axis of coil body 20 respectively. However, the axis lines O and O1 may be different from the central axes of the core shaft 10 and the coil body 20 respectively. Fig. 1 illustrates XYZ-axes orthogonal to each other. The X-axis corresponds to a length direction of the guide wire 1, the Y-axis corresponds to a height direction of the guide wire 1, and the Z-axis corresponds to a width direction of the guide wire 1. In Fig. 1, the left side (-X-axis direction) is referred to as a "distal end side" of the guide wire 1 and each component, and the right side (+X-axis direction) is referred to as a "proximal end side" of the guide wire 1 and each component. As for the guide wire 1 and each component, an end portion positioned on the distal end side is referred to as a "distal end", and the distal end and the vicinity thereof are referred to as a "distal end portion". In addition, an end portion positioned on the proximal end side is referred to as a "proximal end", and the proximal end and the vicinity thereof are referred to as a "proximal end portion". The distal end side corresponds to a "far side" to be inserted into a living body, and the proximal end side corresponds to a "near side" to be operated by an operator such as a surgeon. These definitions are common for the figures following Fig. 1.

The core shaft 10 is a tapered long member having a large diameter on the proximal end side and a small diameter on the distal end side. The core shaft 10 is arranged to extend coaxially with the axis line O1 on the distal end portion and extend coaxially with the axis line O on the proximal end side with respect to the distal end portion. The core shaft 10 can be made of a material, e.g. a stainless alloy such as SUS304 and SUS316, a superelastic alloy such as nickel-titanium (NiTi) alloy, a piano wire, a nickel-chromium alloy, a cobalt alloy, or tungsten, or the like. The core shaft 10 may be made of a known material other than the aforementioned materials. The core shaft 10 includes a distal end portion 11, a first intermediate portion 12, a second intermediate portion 13, an increased diameter portion 14, and a large diameter portion 15, in this order from the distal end side to the proximal end side.

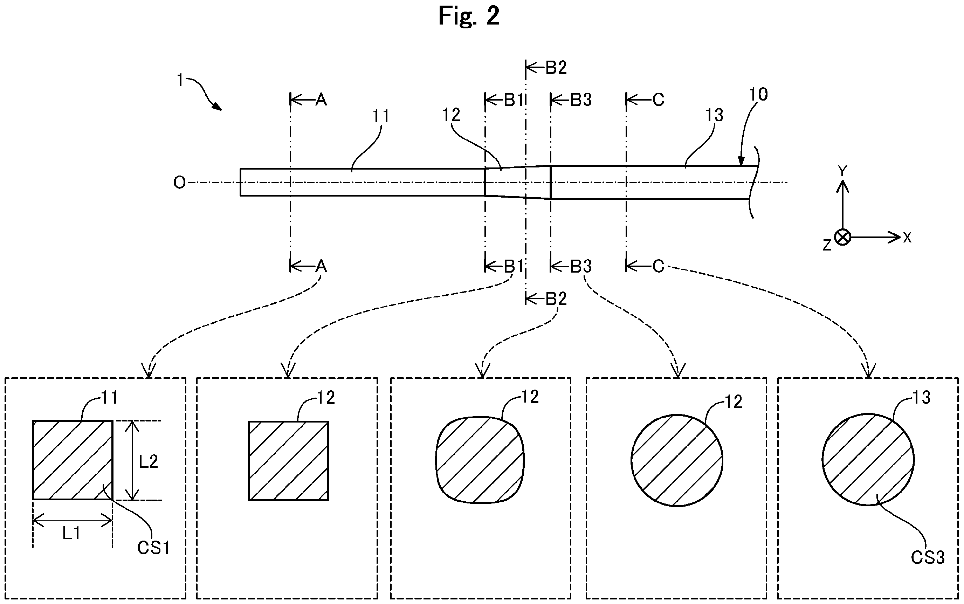

Fig. 2 is an explanatory diagram illustrating a sectional configuration on the distal end side of the core shaft 10. Fig. 2 illustrates the distal end side of the core shaft 10 in an uncurved state for convenience of illustration. The distal end portion 11 is provided on the distal end portion of the core shaft 10. The distal end portion 11 has a prismatic shape with a substantially constant outer diameter, and has a rectangular transverse sectional shape over the entire length (Fig. 2: A-A section). Herein, at any position in the longitudinal direction along the axis line O or the axis line O1, a length in a width direction of the rectangle constituting the transverse section of the distal end portion 11 is defined as L1, and a length in a height direction is defined as L2. At this time, on the distal end portion 11 according to the first embodiment, an aspect ratio of the rectangle constituting the transverse section is greater than or equal to 1:1 and less than or equal to 1.08:1. Herein, the aspect ratio means a ratio between a long side and a short side. In a case of width L1 > height L2, L1:L2 is greater than or equal to 1:1 and less than or equal to 1.08:1, and in a case of width L1 < height L2, L2:L1 is greater than or equal to 1:1 and less than or equal to 1.08:1.

The first intermediate portion 12 is provided adjacent to the proximal end side of the distal end portion 11. The first intermediate portion 12 is a member of which the shape gradually changes from a prismatic shape to a cylindrical shape from the distal end side toward the proximal end side. That means, the distal transverse sectional shape of the first intermediate portion 12 is rectangular (Fig. 2: B1-B1 section), the proximal transverse sectional shape of the first intermediate portion 12 is circular (Fig. 2: B3-B3 section), and the transverse sectional shape between the distal end and the proximal end of the first intermediate portion 12 is a substantially rectangular shape with R at corners (Fig. 2: B2-B2 section). Herein, the corners R between the distal end and the proximal end of the first intermediate portion 12 gradually increase from the distal end side toward the proximal end side. That means, the first intermediate portion 12 has a transverse sectional shape that gradually changes from a rectangular shape to a circular shape from the distal end toward the proximal end.

The second intermediate portion 13 is provided adjacent to the proximal end side of the first intermediate portion 12. The second intermediate portion 13 has a cylindrical shape having a substantially constant outer diameter, and has a circular transverse sectional shape over the entire length (Fig. 2: C-C section). In the core shaft 10 according to the first embodiment, a sectional area CS3 (Fig. 2: C-C section) of the transverse section on the distal end portion of the second intermediate portion 13 is equal to a sectional area CS1 (Fig. 2: A-A section) of the transverse section at any position in the longitudinal direction of the distal end portion 11. Herein, the "equal to" means substantially-equal dimensions, and if the sectional area CS3 and the sectional area CS1 are within a predetermined error range (e.g., within 10%), the sectional area CS3 and the sectional area CS1 are supposed to be equal.

The distal end portion 11, the first intermediate portion 12, and the second intermediate portion 13 can be prepared e.g. as described below. First, a cylindrical member having a substantially constant outer diameter is prepared. Next, a part on the distal end side of the cylindrical member is pressed in a Y-axis direction and then pressed in a Z-axis direction. During the press, the member positioned inside a jig corresponds to the distal end portion 11, the member positioned on an end portion of the jig corresponds to the first intermediate portion 12, and the member positioned outside the jig corresponds to the second intermediate portion 13. Thus, the press makes it easy to prepare the distal end portion 11, the first intermediate portion 12, and the second intermediate portion 13. Note that the distal end portion 11, the first intermediate portion 12, and the second intermediate portion 13 may be separately formed in advance and then welded or joined together.

Fig. 1 will be explained again. The increased diameter portion 14 is provided adjacent to the proximal end side of the second intermediate portion 13. The increased diameter portion 14 has an almost truncated cone shape with an outer diameter gradually increasing from the distal end side toward the proximal end side. The large diameter portion 15 is provided adjacent to the proximal end side of the increased diameter portion 14. The large diameter portion 15 is a portion where the outer diameter of the core shaft 10 is largest, and has an almost cylindrical shape with a substantially constant outer diameter. The outer diameters and lengths of the distal end portion 11, the first intermediate portion 12, the second intermediate portion 13, the increased diameter portion 14, and the large diameter portion 15 can be arbitrarily determined. The distal end portion 11, the first intermediate portion 12, the second intermediate portion 13, the increased diameter portion 14, and the large diameter portion 15 are not necessarily solid, and may be hollow. Shapes of the increased diameter portion 14 and the large diameter portion 15 can be arbitrarily determined, and may be prismatic, polygonal columnar, or the like.

The coil body 20 has an almost hollow cylindrical shape formed by spirally winding a wire 21 around the core shaft 10. In the example of Fig. 1, the coil body 20 covers the distal end portion 11, the first intermediate portion 12, and the second intermediate portion 13 of the core shaft 10. The coil body 20 may be a single-thread coil formed by winding one wire in a single-thread pattern, a multi-thread coil formed by winding a plurality of wires in a multi-thread pattern, a single-thread strand coil formed by winding a strand composed of a plurality of wires twisted together in a single-thread pattern, or a multi-thread strand coil formed by winding, in a multi-thread pattern, a plurality of strands composed of a plurality of wires twisted together. For the coil body 20, a wire diameter of the wire 21 and an average diameter of the coil (an average diameter of the outer diameter and inner diameter of the coil body 20) can be arbitrarily determined.

The wire 21 can be made of a stainless alloy such as SUS304 and SUS316, a superelastic alloy such as NiTi alloy, a piano wire, a radiolucent alloy such as nickel-chromium alloy and cobalt alloy, gold, platinum, tungsten, a radiopaque alloy such as an alloy containing any of these elements (e.g. a platinum-nickel alloy), or another known material.

The strand 30 is composed of a plurality of wires twisted together. The strand 30 is arranged alongside the core shaft 10 inside the coil body 20. As illustrated in Fig. 1, the distal end portion of the strand 30 is disposed at a position corresponding to the distal end of the distal end portion 11 of the core shaft 10, and the proximal end portion of the strand 30 is disposed at a position corresponding to the distal end side of the second intermediate portion 13 of the core shaft 10. A material of the wire constituting the strand 30 may be the same as or different from that of the wire 21. The number of the wires constituting the strand 30 can be arbitrarily determined. In the first embodiment, a diameter of the transverse section at any position in the longitudinal direction of the strand 30 is larger than both the width L1 and the height L2 of the distal end portion 11 of the core shaft 10. A length of the strand 30 can be arbitrarily determined.

The fixation portion 51 is disposed on the proximal end portion of the strand 30. The fixation portion 51 fixes and integrally holds the proximal end portion of the strand 30, a part of the second intermediate portion 13 of the core shaft 10, and a part of the coil body 20. The distal tip 61 is disposed on the distal end portion of the guide wire 1. The distal tip 61 fixes and integrally holds the distal end portion 11 of the core shaft 10, the distal end portion of the coil body 20, and the distal end portion of the strand 30. The proximal fixation portion 62 is disposed on the proximal end portion of the second intermediate portion 13 of the core shaft 10. The proximal fixation portion 62 fixes and integrally holds the second intermediate portion 13 of the core shaft 10 and the proximal end portion of the coil body 20. The fixation portion 51, the distal tip 61, and the proximal fixation portion 62 can be made of any bonding agent e.g. a metal solder such as silver solder, gold solder, zinc, Sn-Ag alloy, and Au-Sn alloy, or an adhesive such as an epoxy adhesive. For the fixation portion 51, the distal tip 61, and the proximal fixation portion 62, same or different Joining agents may be used.

The curved portion 100 is provided on the distal end side of the guide wire 1, where the core shaft 10, the coil body 20, and the strand 30 are curved with respect to the axis line O direction of the guide wire 1. An angle of the curved portion 100 i.e. an angle of the axis line O1 with respect to the axis line O can be arbitrarily determined. The shape of the curved portion 100 may be, besides the angled form illustrated in Fig. 1, any shape such as a J shape, a small J shape, and a swan shape.

### <Example of Effect>

As described above, in the guide wire 1 according to the first embodiment, the transverse sectional shape of the distal end portion 11 of the core shaft 10 is rectangular, specifically an almost square shape with a rectangle aspect ratio of greater than or equal to 1:1 and less than or equal to 1.08:1 (Fig. 2: A-A section). This makes it possible to achieve both the shaping performance and rotation followability of the guide wire 1. In addition, when the rectangle aspect ratio of the distal end portion 11 is set to be greater than or equal to 1:1 and less than or equal to 1.08:1, the shaping performance and the rotation followability of the guide wire 1 can be maintained while facilitating manufacture of the guide wire 1.

In the guide wire 1 according to the first embodiment, the core shaft 10 includes the first intermediate portion 12 having the transverse sectional shape gradually changing from the rectangular shape to the circular shape from the distal end toward the proximal end, between the prismatic (i.e. rectangular transverse sectional shape) distal end portion 11 and the second intermediate portion 13 having the circular transverse sectional shape (Fig. 2: B1-B1 section, B2-B2 section, B3-B3 section). Thereby, it is possible to smoothly change the rigidity and the outer shape from the distal end portion 11, to the first intermediate portion 12, and to the second intermediate portion 13. As a result, stress concentration at a specific position of the core shaft 10 can be suppressed, and kink resistance of the guide wire 1 can be improved during shaping and use of the guide wire 1. In the core shaft 10, the sectional area CS1 of the transverse section on the distal end portion 11 is equal to the sectional area CS3 of the transverse section on the distal end of the second intermediate portion 13. Thereby, stress concentration at a specific position of the core shaft 10 can be suppressed, and kink resistance of the guide wire 1 can be improved during shaping and use of the guide wire 1.

Furthermore, the guide wire 1 according to the first embodiment further includes the strand 30 composed of a plurality of wires twisted together, and therefore can be configured such that the strand 30 and the core shaft 10 integrally move on the distal end side of the guide wire 1 during use of the guide wire 1. As a result, the guide wire 1 excellent in flexibility and restorability against bending can be provided. The curved portion 100 where the strand 30, the core shaft 10, and the coil body 20 are curved is provided on the distal end side of the guide wire 1. As described above, since the distal end portion 11 of the core shaft 10 has the rectangular transverse sectional shape with the aspect ratio of greater than or equal to 1:1 and less than or equal to 1.08:1, the curved portion 100 can be easily provided on the distal end side of the guide wire 1.

### <Rotation Followability Test>

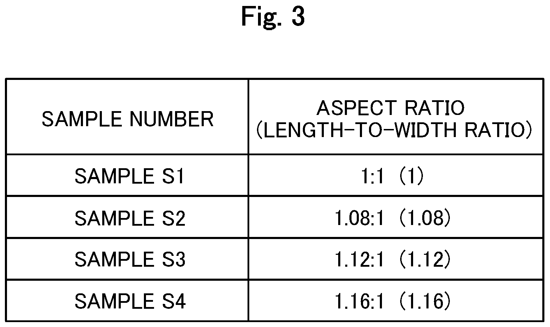

With reference to Fig. 3 to Fig. 5, it will be explained that the rotation followability can be maintained by the guide wire 1 according to the first embodiment. Herein, in order to prove the effectiveness of the guide wire 1 according to the first embodiment, a rotation followability test was conducted for four samples including guide wires having the configuration according to the first embodiment. The rotation followability test refers to a test in which the rotation followability of the guide wire is quantitatively measured.

Fig. 3 is an explanatory diagram illustrating configurations of samples of the guide wires used in the rotation followability test. Samples S1 to S4 include the core shaft 10, the coil body 20, the strand 30, the fixation portion 51, the distal tip 61, and the proximal fixation portion 62, as in the guide wire 1 according to the aforementioned first embodiment (Fig. 1). The samples S1 to S4 differ from each other in the configuration of the distal end portion 11 of the core shaft 10. Specifically, the samples S1 to S4 differ from each other in the aspect ratio between the width L1 and the height L2 of the rectangle constituting the transverse section of the distal end portion 11. Configurations excluding the aspect ratio in the samples S1 to S4 are the same as those of the guide wire 1 explained in the first embodiment.

As illustrated in Fig. 3, the distal end portion of the core shaft in the sample S1 has 1:1 of aspect ratio between the width L1 and the height L2 (length-to-width ratio=1). In addition, the distal end portion of the core shaft in the sample S2 has 1.08:1 of aspect ratio between the width L1 and the height L2 (length-to-width ratio=1.08), the distal end portion of the core shaft in the sample S3 has 1.12:1 of aspect ratio between the width L1 and the height L2 (length-to-width ratio=1.12), and the distal end portion of the core shaft in the sample S4 has 1.16:1 of aspect ratio between the width L1 and the height L2 (length-to-width ratio=1.16).

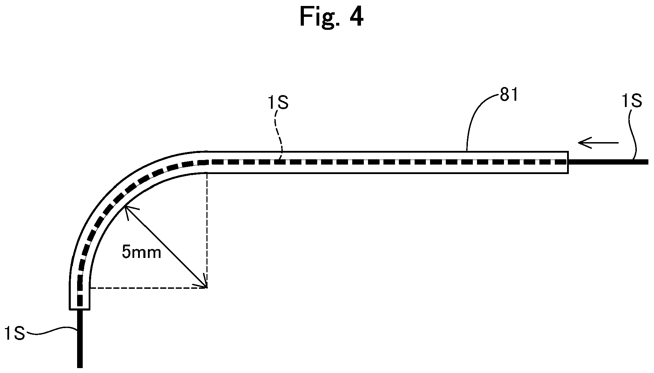

Fig. 4 is an explanatory diagram of a test method for the rotation followability test. In the rotation followability test, a tube 81 is bent at 90° with a radius of 5 mm, and straight portions are formed ahead of and behind the bend, to create a test path. The sample guide wire 1S was inserted into the tube 81 from one opening (the opening on the right side of Fig. 4) of this test path. Subsequently, the sample guide wire 1S was pushed forward to the inner depth until the distal end protruded from the other opening of the tube 81. In this state, the proximal end side of the guide wire 1S was rotated. In the rotation followability test, for each of the aforementioned samples S 1 to S4, the number of rotations on the distal end side in response to the rotation on the proximal end side was measured.

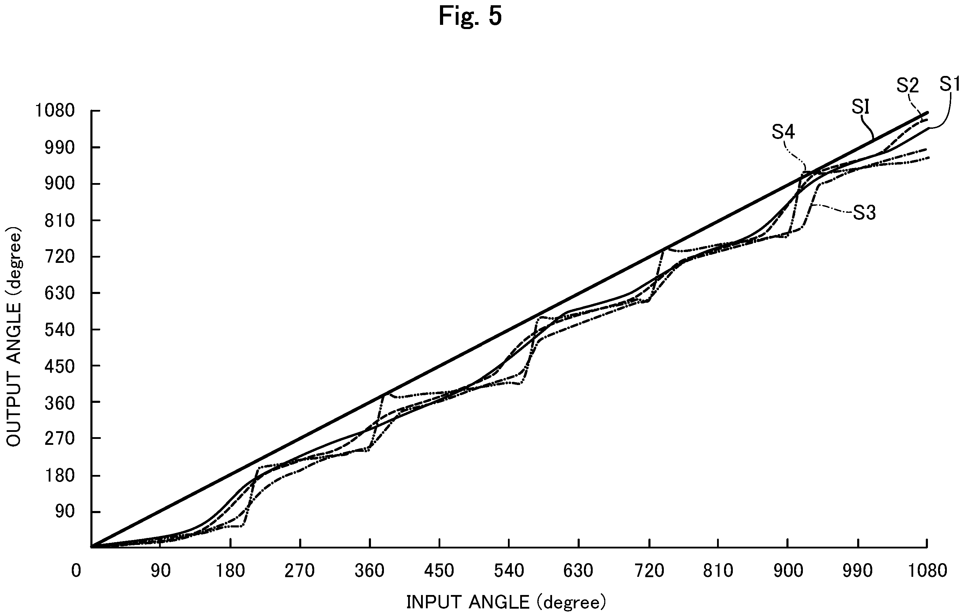

Fig. 5 is an explanatory diagram of a test result for the rotation followability test. In Fig. 5, the abscissa indicates a rotation angle (input angle) of the proximal end side of the sample guide wire 1S, and the ordinate indicates a rotation angle (output angle) of the distal end side of the sample guide wire 1S. In Fig. 5, a measurement result of the sample S1 is represented by a solid line, a measurement result of the sample S2 is represented by a dashed line, a measurement result of the sample S3 is represented by a dot and dash line, and a measurement result of the sample S4 is represented by a two-dot and dash line. Additionally, in Fig. 5, ideal values SI are represented by a bold line. The ideal value SI refers to a state that the distal end side completely follows the rotation of the proximal end side.

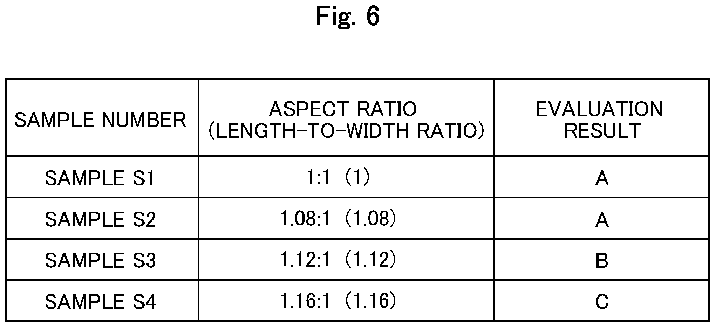

Fig. 6 is an explanatory diagram of evaluation results for the rotation followability test. In the evaluation results illustrated in Fig. 6, ranks A, B, and C are set in descending order of excellence. Both in the sample S1 with 1:1 of aspect ratio and the sample S2 with 1.08:1 of aspect ratio, the output angle gradually follows the input angle, and therefore the samples S1 and S2 have relatively high rotation followability (Fig. 5). Thus, the evaluation results of the rotation followability test of the samples S1 and S2 were ranked as A. On the other hand, in the sample S3 with 1.12:1 of aspect ratio, the followability of the output angle following the input angle is inferior to those of the samples S1 and S2, and therefore it is not considered that the sample S3 has a relatively high rotation followability (Fig. 5). Consequently, the evaluation result of the rotation followability test for the sample S3 was ranked as B. In the sample S4 with 1.16:1 of aspect ratio, the follow of the output angle following the input angle is not gradual but is delayed (Fig. 5). This indicates that the rotation of the proximal end side is not transmitted to the distal end side in real time, and that the torque has accumulated for a while, then the torque is suddenly released, and thereby the distal end side rotates (causing a so-called "springing-up"). Consequently, the evaluation result of the rotation followability test for the sample S4 was ranked as C.

The above rotational followability tests elucidated that the higher the aspect ratio between the width L1 and the height L2 of the rectangle constituting the transverse section of the distal end portion 11 of the core shaft 10, the lower the rotation followability. Also, the rotation followability tests elucidated that the aspect ratio between the width L1 and the height L2 was preferably greater than or equal to 1:1 and less than or equal to 1.08:1.

### <Second Embodiment>

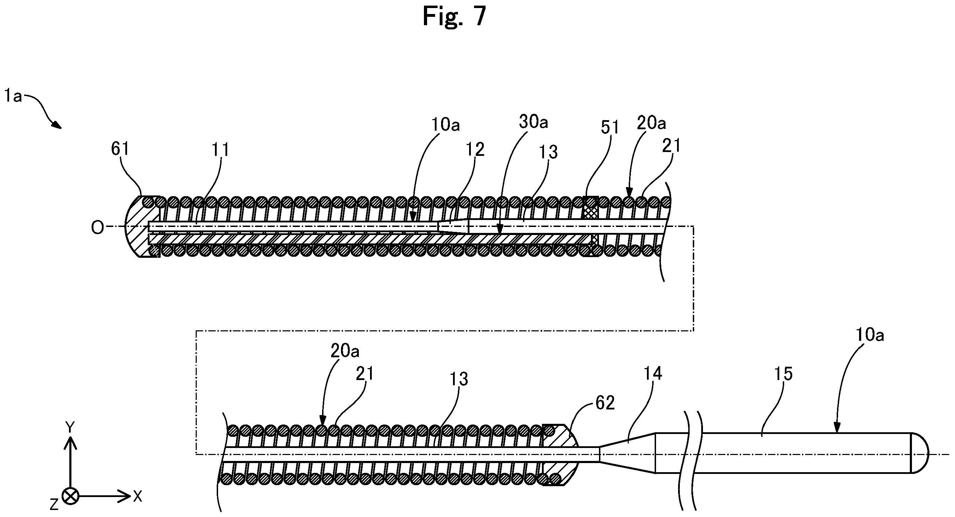

Fig. 7 is an explanatory diagram illustrating a configuration of a guide wire 1a according to the second embodiment. The guide wire 1a according to the second embodiment does not include the curved portion 100 explained in the first embodiment. That means, in the guide wire 1a, a core shaft 10a, a coil body 20a, and a strand 30a are not curved on the distal end side, and are straight over the entire length. Thus, the configuration of the guide wire 1a can be variously modified and may be configured in a straight line without the curved portion 100. Also, this guide wire 1a according to the second embodiment allows an operator to provide a desired curve to the distal end side of the guide wire 1a prior to use of the guide wire 1a, so that the same effect as in the first embodiment can be exhibited.

### <Third Embodiment>

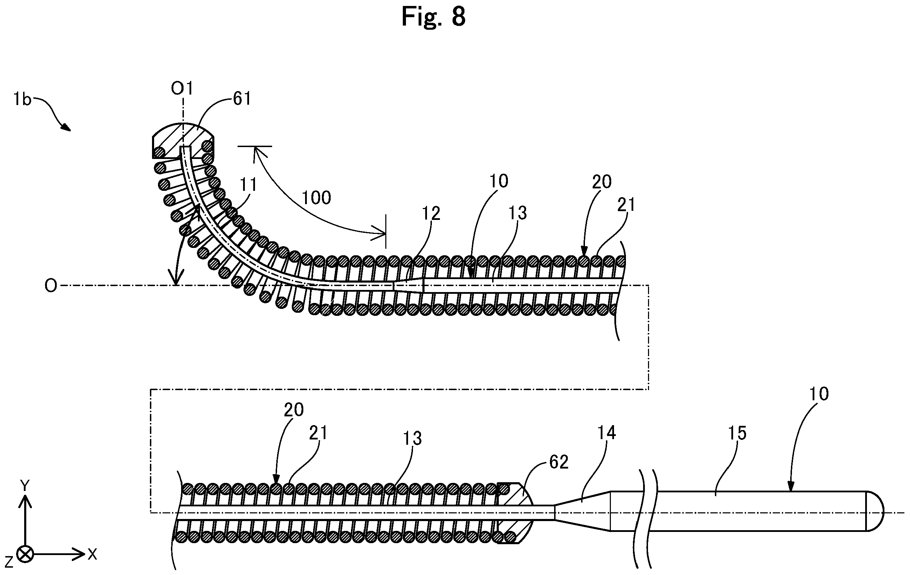

Fig. 8 is an explanatory diagram illustrating a configuration of a guide wire 1b according to the third embodiment. The guide wire 1b according to the third embodiment does not include the strand 30 and the fixation portion 51. Thus, the configuration of the guide wire 1b can be variously modified, and the strand 30 and the fixation portion 51 may be omitted. Also, this guide wire 1b according to the third embodiment can exhibit the same effect as in the first embodiment.

The guide wire 1b may be configured to include an inner covering portion instead of the strand 30, and an inner fixation portion for fixing the inner covering portion, the core shaft 10, and the coil body 20 instead of the fixation portion 51. The inner covering portion is disposed inside the coil body 20, such that the distal end portion is fixed by the distal tip 61 and the proximal end portion is fixed by the inner fixation portion. As the inner covering portion, for example, a coil body formed by spirally winding a wire around the core shaft 10 or a tubular body for covering the periphery of the core shaft 10 can be adopted. The inner covering portion can be disposed at any position in the axis line O direction. For example, when the inner covering portion is provided at a position that covers the distal end portion 11 of the core shaft 10, flexibility and durability on the distal end portion of the guide wire 1b can be improved.

### <Fourth Embodiment>

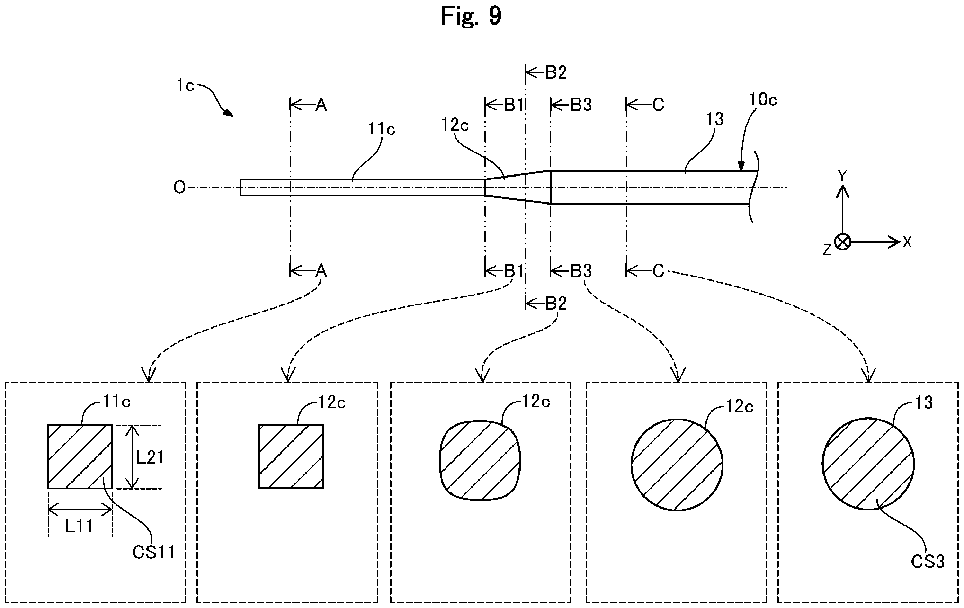

Fig. 9 is an explanatory diagram illustrating a sectional configuration on a distal end side of a core shaft 10c according to the fourth embodiment. The guide wire 1c according to the fourth embodiment includes the core shaft 10c instead of the core shaft 10 according to the first embodiment. The core shaft 10c includes a distal end portion 11c instead of the distal end portion 11, and a first intermediate portion 12c instead of the first intermediate portion 12.

The distal end portion 11c is a part where an outer diameter of the core shaft 10c is smallest, and a width L11 and a height L21 of a rectangle constituting the transverse section (Fig. 9: A-A section) are both smaller than the width L1 and the height L2 respectively, of the distal end portion 11 according to the first embodiment (Fig. 2: A-A section). Similarly to the first embodiment, an aspect ratio of the rectangle constituting the transverse section of the distal end portion 11c is greater than or equal to 1:1 and less than or equal to 1.08:1. The first intermediate portion 12c is provided adjacent to the proximal end side of the distal end portion 11c, where the outer diameter gradually increases from the distal end side toward the proximal end side. Similarly to the first embodiment, the first intermediate portion 12c has a transverse sectional shape that gradually changes from a rectangular shape (Fig. 9: B1-B1 section) to a circular shape (Fig. 9: B3-B3 section) from the distal end toward the proximal end. In the core shaft 10c according to the fourth embodiment, the sectional area CS3 (Fig. 9: C-C section) of the transverse section on the distal end portion of the second intermediate portion 13 is larger than a sectional area CS11 (Fig. 9: A-A section) of the transverse section at any position in the longitudinal direction of the distal end portion 11c.

The distal end portion 11c, the first intermediate portion 12c, and the second intermediate portion 13 can be prepared e.g. as described below. First, a cylindrical member having a substantially constant outer diameter is prepared. Next, a part on the distal end side of the cylindrical member is cut out to form the distal end portion 11c and the first intermediate portion 12c. Note that the distal end portion 11c, the first intermediate portion 12c, and the second intermediate portion 13 may be separately formed in advance and then welded or joined together.

As described above, the configuration of the core shaft 10c can be variously modified, and the core shaft 10c may be configured such that the diameter of the distal end portion 11 is smaller than of the second intermediate portion 13, and the sectional area CS3 of the transverse section of the second intermediate portion 13 differs from the sectional area CS11 of the transverse section of the distal end portion 11c. Also, this guide wire 1c according to the fourth embodiment can exhibit the same effect as in the aforementioned first embodiment. Furthermore, in the guide wire 1c according to the fourth embodiment, the core shaft 10c can be gradually decreased in diameter from the second intermediate portion 13 on the proximal end side toward the distal end portion 11c on the distal end side, so that the distal end portion of the guide wire 1c can be made more flexible.

### <Fifth Embodiment>

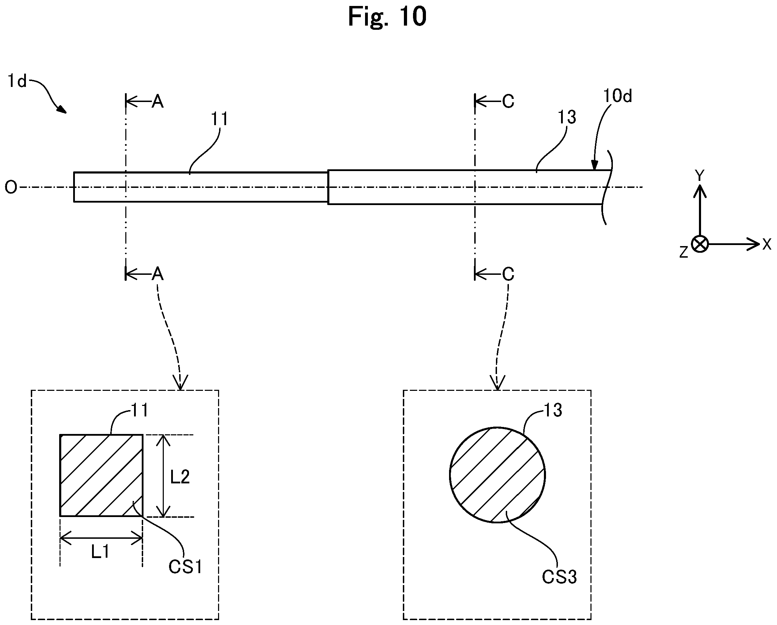

Fig. 10 is an explanatory diagram illustrating a sectional configuration on a distal end side of a core shaft 10d according to the fifth embodiment. A guide wire 1d according to the fifth embodiment includes the core shaft 10d instead of the core shaft 10 according to the first embodiment. The core shaft 10d does not include the first intermediate portion 12 explained in the first embodiment, and the second intermediate portion 13 is provided adjacent to the proximal end side of the distal end portion 11. Thus, the configuration of the core shaft 10d can be variously modified, and the first intermediate portion 12 may be omitted as illustrated in the figure, or the second intermediate portion 13 may be omitted. When the second intermediate portion 13 is omitted, the increased diameter portion 14 (Fig. 1) is provided adjacent to the proximal end side of the distal end portion 11, and the proximal fixation portion 62 (Fig. 1) is provided on the proximal end portion of the distal end portion 11. In addition, the first intermediate portion may have a prismatic shape larger than the distal end portion. Thus, the configuration of the core shaft 10d can be variously modified, and the first intermediate portion 12 or the second intermediate portion 13 may be omitted. Also, this guide wire 1d according to the fifth embodiment can exhibit the same effect as in the aforementioned first embodiment.

### <Sixth Embodiment>

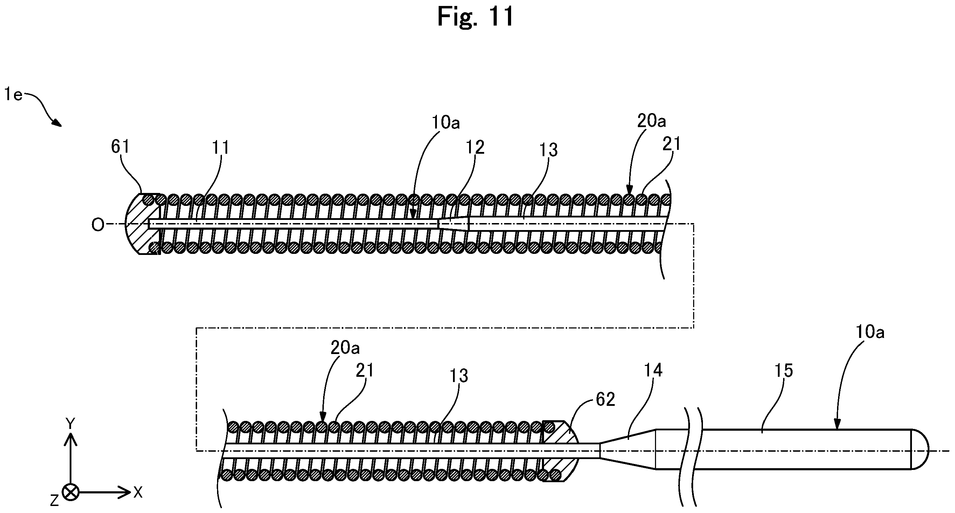

Fig. 11 is an explanatory diagram illustrating a configuration of a guide wire 1e according to the sixth embodiment. The guide wire 1e according to the sixth embodiment does not include the strand 30 and the fixation portion 51 in the configuration explained in the second embodiment. Thus, the configuration of the guide wire 1e can be variously modified, and the curved portion 100, the strand 30, and the fixation portion 51 may be omitted. Also, this guide wire 1e according to the sixth embodiment can exhibit the same effect as in the aforementioned first embodiment.

### <Seventh Embodiment>

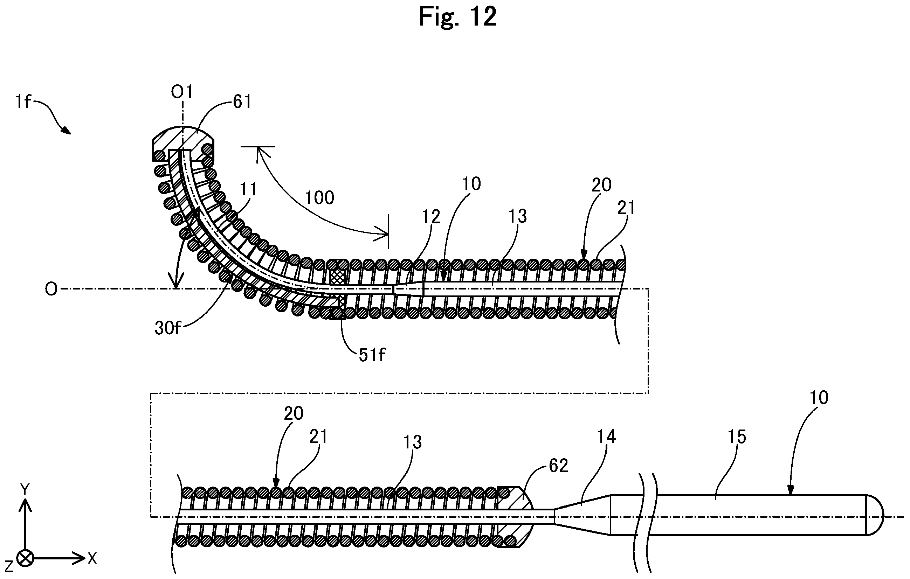

Fig. 12 is an explanatory diagram illustrating a configuration of a guide wire 1f according to the seventh embodiment. The guide wire 1f according to the seventh embodiment includes a strand 30f instead of the strand 30 according to the first embodiment, and a fixation portion 51f instead of the fixation portion 51 according to the first embodiment. The

strand 30f is composed of a plurality of wires twisted together, and arranged along with the core shaft 10 inside the coil body 20. The distal end portion of the strand 30f is disposed at a position corresponding to the distal end of the distal end portion 11 of the core shaft 10, and the proximal end portion of the strand 30f is disposed at a position corresponding to the proximal end side of the distal end portion 11 of the core shaft 10. The fixation portion 51f fixes and integrally holds the proximal end portion of the strand 30f, a part of the distal end portion 11 of the core shaft 10, and a part of the coil body 20.

Thus, the configuration of the strand 30f can be variously modified, and the length and arrangement in the axis line O direction may be arbitrarily changed. For example, the strand 30f may be configured to extend from the distal end to the proximal end of the distal end portion 11 as illustrated in the figure. The strand 30f may be configured to extend from the distal end of the distal end portion 11 to the first intermediate portion 12, or from the distal end of the distal end portion 11 to the proximal end portion of the second intermediate portion 13. When the strand 30f is configured to extend from the distal end of the distal end portion 11 to the proximal end portion of the second intermediate portion 13, the fixation portion 51f may be omitted by fixing the distal end portion of the strand 30f using the distal tip 61 and fixing the proximal end portion using the proximal fixation portion 62. The distal end portion of the strand 30f may be disposed at a position other than the distal end of the distal end portion 11 (e.g. at the middle of the distal end portion 11, or the first intermediate portion 12). In this case, a second fixation portion for fixing the distal end portion of the strand 30f, the core shaft 10, and the coil body 20 may be newly provided. Also, this guide wire 1f according to the seventh embodiment can exhibit the same effect as in the aforementioned first embodiment.

### <Modification Examples of the Embodiments>

The disclosed embodiments are not limited to the above embodiments, and may be implemented in various modes without departing from the gist of the disclosed embodiments. For example, the following modifications can also be applied.

### [Modification Example 1]

In the first to seventh embodiments, the configurations of the guide wires 1 and 1a to 1f have been illustrated. However, the configuration of the guide wire can be variously modified. For example, the guide wires according to the above embodiments have been explained as medical devices used for inserting a catheter into a blood vessel, but the guide wire may be configured to be inserted into various organs in a human body, such as a lymphatic system, a biliary system, a urinary system, a respiratory system, a digestive system, a secretory gland, and reproductive organs. For example, the guide wire may be configured not to include the increased diameter portion and the large diameter portion, and configured such that the whole core shaft is covered with the coil body.

### [Modification Example 2]

In the first to seventh embodiments, the configurations of the core shafts 10, 10a, 10c, and 10d have been illustrated. However, the configuration of the core shaft can be variously modified. For example, the core shaft may be configured such that the distal end portion, the first intermediate portion, the second intermediate portion, the increased diameter portion, and the large diameter portion are not distinguished from each other, and all of the portions constitute a main body portion having a constant outer diameter, or all of the portions constitute a reduced diameter portion having an outer diameter gradually decreasing from the distal end side toward the distal end side. For example, the core shaft may be configured such that the distal end portion, the first intermediate portion, the second intermediate portion, the increased diameter portion, the large diameter portion are made of different materials, and then these portions are joined together. For example, the transverse sectional shapes of the first intermediate portion, the second intermediate portion, the increased diameter portion, the large diameter portion of the core shaft are not limited to the almost circular shape, and, for example, any shape such as an almost rectangular shape, an almost elliptical shape, and an almost polygonal shape can be adopted. For example, on a proximal end side with respect to the large diameter portion, the core shaft may be composed of a plurality of core shaft members joined together. In this case, the core shaft members may be made of the same material or different materials.

### [Modification Example 3]

In the first to seventh embodiments, examples of the configurations of the coil bodies 20 and 20a and the strands 30, 30a, and 30f have been described. However, the configurations of the coil body and the strand can be variously modified. For example, the coil body may have a dense winding configuration with no gap between adjacent wires, a coarse winding configuration with gaps between adjacent wires, or a configuration in which the dense winding and the coarse winding are combined. The coil body may include, for example, a resin layer coated with a hydrophobic resin material, a hydrophilic resin material, or a mixture thereof. For example, the strand may have a configuration in which a plurality of wires are twisted together to form a strand, and a plurality of the strands are further twisted together to form a strand. For example, the wire of at least one of the coil body and the strand does not necessarily have an almost circular transverse sectional shape.

### [Modification Example 4]

The configurations of the guide wires 1 and 1a to 1f according to the first to seventh embodiments, and the configurations of the guide wires according to the first to third modification examples may be appropriately combined. For example, any of the guide wire 1a according to the second embodiment (configuration having no curved portion), the guide wire 1b according to the third embodiment (configuration having no strand), the guide wire 1e according to the sixth embodiment (configuration having no curved portion and no strand), and the guide wire 1f according to the seventh embodiment (configuration having strands with different forms) may include the core shaft explained in the fourth or fifth embodiments.

Although the aspects of the disclosed embodiments have been explained above based on the embodiments and the modification examples, the embodiments of the aforementioned aspects are made for facilitating understanding of the aspects, and do not limit the aspects. The aspects can be modified and improved without departing from the spirit of the aspects and the scope of claims, and the aspects include equivalents thereof. Further, unless the technical features are described as essential in the present specification, the technical features may be omitted as appropriate.

### DESCRIPTION OF REFERENCE NUMERALS

- 1, 1a to 1f: Guide wire
- 10, 10a, 10c, 10d: Core shaft
- 11, 11c: Distal end portion
- 12, 12c: First intermediate portion
- 13: Second intermediate portion
- 14: Increased diameter portion
- 15: Large diameter portion
- 20, 20a: Coil body
- 30, 30a, 30f: Strand
- 51, 51f: Fixation portion
- 61: Distal tip
- 62: Proximal fixation portion
- 81: Tube
- 100: Curved portion

## Claims

1. A guide wire comprising:
a core shaft;
a coil body wound around a distal end side of the core shaft; and
a distal tip to which a distal end portion of the core shaft and a distal end portion of the coil body are fixed, wherein
the distal end portion of the core shaft has a rectangular transverse sectional shape, and an aspect ratio of the rectangle is greater than or equal to 1:1 and less than or equal to 1.08:1.

2. The guide wire according to claim 1, wherein
the core shaft comprises:
the distal end portion having a prismatic shape;
a first intermediate portion adjacent to a proximal end side of the distal end portion and having a rectangular transverse sectional shape on the distal end and a circular transverse sectional shape on the proximal end in which the transverse sectional shape gradually changes from the rectangular shape to the circular shape from the distal end toward the proximal end; and
a second intermediate portion adjacent to the proximal end side of the first intermediate portion and having a circular transverse sectional shape over an entire length thereof.

3. The guide wire according to claim 2, wherein
a sectional area of a transverse section at any position of the distal end portion is equal to a sectional area of a transverse section on a distal end of the second intermediate portion.

4. The guide wire according to any one of claims 1 to 3, wherein
a curved portion in which the core shaft and the coil body are curved with respect to an axial direction of the guide wire is provided on a distal end side of the guide wire.

5. The guide wire according to any one of claims 1 to 3, further comprising:
a strand composed of a plurality of wires twisted together, which is arranged alongside the core shaft inside the coil body, and whose distal end portion fixed to the distal tip; and
a fixation portion provided on the second intermediate portion of the core shaft, to which a proximal end portion of the strand, the core shaft, and the coil body are fixed.

6. The guide wire according to claim 5, wherein
a curved portion in which the strand, the core shaft, and the coil body are curved with respect to the axial direction of the guide wire is provided on a distal end side of the guide wire.
